# EUROPÄISCHE PATENTSCHRIFT

(11) **EP 1 017 663 B1**
(45) Veröffentlichungstag und Bekanntmachung des Hinweises auf die Patenterteilung: **12.06.2002**
(21) Anmeldenummer: 98947523.1
(22) Anmeldetag: 05.09.1998
(51) Int. Cl.: C07C 51/367

(54) **VERFAHREN ZUR HERSTELLUNG VON HELLFARBIGEN ETHERCARBONSÄUREN**
METHOD FOR PREPARING LIGHT-COLOURED ETHER CARBOXYLIC ACIDS
PROCEDE DE PREPARATION D'ACIDES CARBOXYLIQUES D'ETHER DE COULEUR CLAIRE

(30) Priorität: 17.09.1997 DE 19740954
(43) Veröffentlichungstag der Anmeldung: 12.07.2000
(73) Patentinhaber: Cognis Deutschland GmbH & Co. KG, 40589 Düsseldorf (DE)
(72) Erfinder: LAGARDEN, Martin, D-40591 Düsseldorf (DE); BEHLER, Ansgar, D-46240 Bottrop (DE)
(86) Internationale Anmeldenummer: EP9805635
(87) Internationale Veröffentlichungsnummer: WO9914180

(56) Entgegenhaltungen:
- EP-A- 0 399 751
- DE-A- 4 224 362
- CHEMICAL ABSTRACTS, vol. 126, no. 3, 20. Januar 1997 Columbus, Ohio, US; abstract no. 31088j, Seite 555; XP002090793 & JP 08 253446 A (KAO CORP, JAPAN)

## Beschreibung

Die Erfindung betrifft ein Verfahren zur Herstellung von Ethercarbonsäuren durch Carboxymethylierung von Alkoholethoxylaten mit Halogenverbindungen in Gegenwart von Reduktionsmitteln.

### Stand der Technik

Ethercarbonsäuren bzw. deren Salze, die von diesem Begriff stets mitumschlossen werden, stellen milde, schaumstarke Aniontenside dar, die beispielsweise in der Kosmetik wie auch in der Textiltechnik Anwendung finden. Eine Übersicht hierzu findet sich beispielsweise von G.Czichocki et al. in **Fat Sci.Technol. 94, 66 (1992).** Zu ihrer Herstellung geht man üblicherweise von Alkoholethoxylaten aus, deren endständige Ethylenoxideinheiten entweder einer katalytischen Oxidation unterworfen oder mit Halogencarbonsäuren bzw. deren Salzen carboxymethyliert werden ([vgl. **Tens.Surf.Det. 29, 169 (1992)**]. Wegen des hohen technischen Aufwandes hat sich das schonende katalytische Verfahren bislang jedoch nicht durchgesetzt. Im Verlauf der Route über die Halogenverbindungen besteht indes das Problem, daß Produkte erhalten werden, die keine ausreichende Farbqualität aufweisen und daher nachträglich aufgehellt werden müssen.

Die Dokumente EP-A-0 399751 und DE-A-4 224 362 offenbaren weitere Verfahren zur Herstellung von Ethercarbonsäuren. Das Dokument Chem. Abstr., Vol.126, no. 3, 1997, Abstr. Nr.: 31088 j, welches ein Abstract ist aus JP-A-8 253 446, offenbart die Herstellung von Hellfarbigen Amino gruppe-enthatenden Fettsäuren, in welches Verfahren NaBH₄ zur Anwendung kommt. Die Aufgabe der vorliegenden Erfindung hat somit darin bestanden, ein verbessertes Verfahren zur Carboxymethylierung von Alkoholethoxylaten mit Halogencarbonsäuren zur Verfügung zu stellen, bei dem Ethercarbonsäuren bzw. deren Salze mit verbesserter Farbqualität resultieren.

### Beschreibung der Erfindung

Gegenstand der Erfindung ist ein Verfahren zur Herstellung von hellfarbigen Ethercarbonsäuren bzw. deren Salzen der Formel **(I)**,

**R**^{**1**}**(OCH**_{**2**}**CH**_{**2**}**)**_{**n**}**OCH**_{**2**}**COOX (I)**

in der R¹ für einen linearen oder verzweigten Alkyl-, Alkenyl- und/oder Alkylphenylrest mit 6 bis 22 Kohlenstofatomen, X für Wasserstoff oder ein Alkalimetall und n für Zahlen von 1 bis 10 steht, durch Carboxymethylierung von Alkohol- bzw. Alkylphenolethoxylaten der Formel **(II)**,

**R**^{**1**}**(OCH**_{**2**}**CH**_{**2**}**)**_{**n**}**OX (II)**

in der R¹, X und n die obigen Bedeutungen haben, mit Halogenessigsäure oder deren Alkalisalzen, welches sich dadurch auszeichnet, daß man die Reaktion in Gegenwart von Reduktionsmitteln durchführt.

Überraschenderweise wurde gefunden, daß unter Mitverwendung schon von sehr geringen Mengen an Reduktionsmitteln, wie beispielsweise Natriumboranat oder Phosphinsäure, Ethercarbonsäuren bzw. Ethercarbonsäuresalze erhalten werden, die sich durch eine deutlich bessere Farbqualität auszeichnen.

### Alkohol- bzw. Alkylphenolethoxylate

Alkoholethoxylate, die als Ausgangsstoffe in Betracht kommen, stellen Additionsprodukte von Ethylenoxid an primäre Alkohole, vorzugsweise Fettalkohole, Oxoalkohole oder deren Gemische dar, die nach den einschlägigen Verfahren der präparativen organischen Chemie erhalten werden können und großtechnisch zur Verfügung stehen. Die Ethoxylate können über den Weg saurer, vorzugsweise aber basischer Katalyse hergestellt werden und dementsprechend eine konventionell breite oder aber eine eingeengte Homologenverteilung aufweisen. Typische Beispiele sind Additionsprodukte von durchschnittlich 1 bis 10 und vorzugsweise 5 bis 9 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Vorzugsweise setzt man Alkoholethoxylate der Formel **(II)** ein, in der R¹ für einen Alkylrest mit 12 bis 18 Kohlenstoffatomen, X für Wasserstoff oder Natrium und n für Zahlen von 1 bis 5 steht, d.h. anstelle der echten Ethoxylate können auch direkt deren basische Alkoholatsalze verwendet werden. Des weiteren können auch Alkylphenolethoxylate der Formel **(II)** eingesetzt werden, in der R¹ vorzugsweise für einen Alkyphenylrest mit 6 bis 9 Kohlenstoffatomen im Alkylrest, X für Wasserstoff oder Natrium und n für Zahlen von 5 bis 7 steht. Typisches Beispiel ist ein Additionsprodukt von durchschnittlich 5 bis 7 Mol Ethylenoxid an Nonylphenol.

### Carboxymethylierungsmittel

Als Carboxymethylierungsmittel können neben der Chlor- bzw. Bromessigsäure auch deren Alkalisalze, vorzugsweise Natrium- oder Kaliumchloracetat, eingesetzt werden. Das molare Einsatzverhältnis zwischen Ethoxylat und Carboxymethylierungsmittel kann dabei 1 : 0,2 bis 1 : 1,2 betragen und liegt vorzugsweise bei 1 : 0,25 bis 1 : 0,6. Sofern mit unterstöchiometrischen Mengen an Carboxymethylierungsmitteln gearbeitet wird, verbleiben im erhaltenen Reaktionsprodukt noch Mengen an unumgesetztem Ethoxylat, die aber nicht störend sind.

### Reduktionsmittel

Als Reduktionsmittel kommen Hydridverbindungen, wie Lithiumaluminiumhydrid (Lithiumalanat), vorzugsweise aber Natriumborhydrid (Natriumboranat) oder Phosphorverbindungen in der Oxidationsstufe (+1), wie beispielsweise Phosphinsäure oder deren Alkalisalze in Frage. Die Reduktionsmittel sind schon in kleinsten Mengen wirksam, d.h. die Einsatzmenge kann - bezogen auf die Einsatzstoffe - 0,1 bis 0,00001, vorzugsweise 0,01 bis 0,0001 und bevorzugt 0,001 bis 0,005 Gew.-% betragen.

### Carboxymethylierung

Die Carboxymethylierung kann in an sich bekannter Weise durchgeführt werden, wobei sich insbesondere bei Einsatz von Hydriden als Reduktionsmittel der Ausschluß von Luftsauerstoff, beispielsweise durch eine Stickstoffabdeckung empfiehlt. Die Reaktion wird in Gegenwart von Basen durchgeführt, wodurch aus dem Ethoxylat das Alkoholat gebildet wird, welches alleine zur Reaktion mit der Halogencarbonsäure bzw. deren Salz befähigt ist. Die Basenmenge ist dabei so zu wählen, daß sie zur Alkoholatbildung ausreicht. Es ist natürlich auch möglich, anstelle der Ethoxylate direkt deren Alkoholate einzusetzen, auch wenn für die erste Alternative spricht, daß sie vom technischen Aufwand her betrachtet, deutlich einfacher ist. Als Basen kommen insbesondere Natrium- und Kaliumhydroxid oder deren Mischungen in Betracht. Sofern eine möglichst vollständige Carboxymethylierung gewünscht wird, ist es von Vorteil, die Ethoxylate und das Carboxymethylierungsmittel vorzulegen und die Basen portionsweise zuzugeben. Sofern keine vollständige Carboxymethylierung angestrebt wird, werden vorzugsweise die Ethoxylate mit den Basen vorgelegt und das Carboxymethylierungsmittel portionsweise zugegeben. Die Reaktionstemperatur liegt in der Regel bei 40 bis 50°C, wobei es sich empfiehlt, die Exothermie bei der Bildung des Alkoholats auszunützen. Temperaturen oberhalb von 50°C führen zwar zu einer Beschleunigung der Reaktion, sind jedoch weniger wünschenswert, da oberhalb einer kritischen Temperatur von 53°C Verfärbungen der Produkte beobachtet werden.

### Beispiele

**Beispiel 1.** In einem 2-l-Dreihalskolben mit Rührer, Kühler, Gaseinleitungsrohr und Innenthermometer wurden bei 20°C in einer Stickstoffatmosphäre 798 g (1,64 mol) Isotridecylalkohol+7EO und 0,0133 g (0,35 mmol) Natriumborhydrid als Reduktionsmittel vorgelegt. Unter intensivem Rühren wurden 22,9 g (0,57 mol) pulverförmiges Natriumhydroxid zugegeben, wobei die Temperatur innerhalb von 15 min auf 30°C anstieg. Anschließend wurden portionsweise innerhalb von 15 min 66,7 g (0,57 mol) Natriumchloracetat zugegeben. Der Ansatz wurde 5 h bei 50°C gerührt, wobei 848 g einer weißen Suspension erhalten wurde. Der Chloridgehalt betrug nach pötentiometrischer Titration 2,01 Gew.-%, entsprechend einem Umsetzungsgrad von 88 % der Theorie. Die Lovibond-Farbmessung in der 5 1/4-Zoll-Küvette ergab: 0,2 blau/0,1 rot/1,0 gelb.

**Beispiel 2.** Beispiel 1 wurde unter Einsatz von 1,1 g (8,3 mmol) Phosphinsäure als Reduktionsmittel wiederholt. Es wurde eine weiße Suspension mit einem Chloridgehalt von 2,19 Gew.-%, entsprechend einem Umsatz von 96 % der Theorie erhalten. Die Lovibond-Farbmessung in der 5 1/4-Zoll-Küvette ergab: 0,9 rot/6,0 gelb (blau nicht bestimmt).

**Vergleichsbeispiel V1.** Beispiel 1 wurde wiederholt, jedoch kein Reduktionsmittel zugesetzt. Es wurde eine rötlich-gelbe Suspension erhalten. Der Chloridgehalt betrug 2,26 Gew.-% entsprechend einem Umsatz von 98 % der Theorie. Die Lovibond-Farbmessung in der 5 1/4-Zoll-Küvette ergab: 5,0 rot/11,0 gelb (blau nicht bestimmt).

## Patentansprüche

1. Verfahren zur Herstellung von hellfarbigen Ethercarbonsäuren bzw. deren Salze der Formel **(I)**,
**R**^{**1**}**(OCH**_{**2**}**CH**_{**2**}**)**_{**n**}**OCH**_{**2**}**COOX** **(I)**
in der R¹ für einen linearen oder verzweigten Alkyl-, Alkenyl- und/oder Alkylphenylrest mit 6 bis 22 Kohlenstoffatomen, X für Wasserstoff oder ein Alkalimetall und n für Zahlen von 1 bis 10 steht, durch Carboxymethylierung von Alkohol- bzw. Alkylphenolethoxylaten der Formel **(II)**,
**R**^{**1**}**(OCH**_{**2**}**CH**_{**2**}**)**_{**n**}**OX** **(II)**
in der R¹, X und n die obigen Bedeutungen haben, mit Halogenessigsäure oder deren Alkalisalzen, **dadurch gekennzeichnet, daß** man die Reaktion in Gegenwart von Reduktionsmitteln durchführt.

2. Verfahren nach Anspruch 1, **dadurch gekennzeichnet, daß** man Alkoholethoxylate der Formel **(II)** einsetzt, in der R¹ für einen Alkylrest mit 12 bis 18 Kohlenstoffatomen, X für Wasserstoff oder Natrium und n für Zahlen von 1 bis 5 steht.

3. Verfahren nach Anspruch 1, **dadurch gekennzeichnet, daß** man Alkylphenolethoxylate der Formel **(II)** einsetzt, in der R¹ für einen Alkylrest mit 6 bis 9 Kohlenstoffatomen, X für Wasserstoff oder Natrium und n für Zahlen von 5 bis 7 steht.

4. Verfahren nach den Ansprüchen 1 bis 3, **dadurch gekennzeichnet, daß** man als Carboxymethylierungsmittel Natriumchloracetat einsetzt.

5. Verfahren nach den Ansprüchen 1 bis 4, **dadurch gekennzeichnet, daß** man als Reduktionsmittel Natriumboranat und/oder Phosphinsäure einsetzt.

6. Verfahren nach den Ansprüchen 1 bis 5, **dadurch gekennzeichnet, daß** man die Reduktionsmittel - bezogen auf die Einsatzstoffe - in Mengen von 0,1 bis 0,00001 Gew.-% einsetzt.

## Claims

1. A process for the production of light-coloured ethercarboxylic acids or salts thereof corresponding to formula **(I)**:
**R**^{**1**}**(OCH**_{**2**}**CH**_{**2**}**)**_{**n**}**OCH**_{**2**}**COOX (I)**
in which R¹ is a linear or branched alkyl, alkenyl and/or alkylphenyl group containing 6 to 22 carbon atoms, X is hydrogen or an alkali metal and n is a number of 1 to 10,
by carboxymethylation of alcohol or alkyl phenolethoxylates corresponding to formula **(II)**:
**R**^{**1**}**(OCH**_{**2**}**CH**_{**2**}**)**_{**n**}**OX (II)**
in which R¹, X and n are as defined above,
with haloacetic acid or alkali metal salts thereof, **characterized in that** the reaction is carried out in the presence of reducing agents.

2. A process as claimed in claim 1, **characterized in that** alcohol ethoxylates corresponding to formula **(II)**, in which R¹ is an alkyl group containing 12 to 18 carbon atoms, X is hydrogen or sodium and n is a number of 1 to 5, are used.

3. A process as claimed in claim 1, **characterized in that** alkylphenol ethoxylates corresponding to formula **(II)**, in which R¹ is an alkyl group containing 6 to 9 carbon atoms, X is hydrogen or sodium and n is a number of 5 to 7, are used.

4. A process as claimed in claims 1 to 3, **characterized in that** sodium chloroacetate is used as the carboxymethylating agent.

5. A process as claimed in claims 1 to 4, **characterized in that** sodium boranate and/or phosphinic acid is/are used as reducing agent(s).

6. A process as claimed in claims 1 to 5, **characterized in that** the reducing agents are used in quantities of 0.1 to 0.00001% by weight, based on the starting materials.

## Revendications

1. Procédé pour la préparation d'acides éthercarboxyliques de couleur claire ou de leurs sels de formule (I),
R¹(OCH₂CH₂)ₙOCH₂COOX (I)
dans laquelle R¹ représente un radical alkyle, alcényle et/ou alkylphényle linéaire ou ramifié, ayant de 6 à 22 atomes de carbone, X représente un atome d'hydrogène ou un métal alcalin, et n représente des nombres allant de 1 à 10,
par carboxylation de produits d'oxyéthylation d'alcools ou d'alkylphénols, de formule (II),
R¹(OCH₂CH₂)ₙOX (II)
dans laquelle R¹, X et n ont les significations données ci-dessus, avec un acide halogénoacétique ou ses sels alcalins, qui se distingue par le fait qu'on effectue la réaction en présence de réducteurs.

2. Procédé selon la revendication 1,
**caractérisé en ce qu'**
on utilise des produits d'oxyéthylation d'alcools de formule (II), dans laquelle R¹ représente un radical alkyle ayant de 12 à 18 atomes de carbone, X représente un atome d'hydrogène ou de sodium et n représente les nombres allant de 1 à 5.

3. Procédé selon la revendication 1,
**caractérisé en ce qu'**
on utilise des produits d'oxyéthylation d'alkylphénols de formule (II), dans laquelle R¹ représente un radical alkyle ayant de 6 à 9 atomes de carbone, X représente un atome d'hydrogène ou de sodium et n représente les nombres allant de 5 à 7.

4. Procédé selon les revendications 1 à 3,
**caractérisé en ce qu'**
on utilise comme agent de carboxylation le chloracétate de sodium.

5. Procédé selon les revendications 1 à 4,
**caractérisé en ce qu'**
on utilise comme réducteur le boranate de sodium et/ou l'acide phosphinique.

6. Procédé selon les revendications 1 à 5,
**caractérisé en ce qu'**
on utilise le réducteur - par rapport aux produits de départ - en quantités de 0,1 à 0,00001 % en poids.
